(19) Europäisches Patentamt
European Patent Office
Office européen des brevets

(11) Veröffentlichungsnummer: **0 019 090**
**B1**

(12) # EUROPÄISCHE PATENTSCHRIFT

(45) Veröffentlichungstag der Patentschrift: **18.05.83**

(21) Anmeldenummer: **80102002.5**

(22) Anmeldetag: **14.04.80**

(51) Int. Cl.³: **C 07 C 69/757,** C 07 C 149/40, C 07 C 62/34, A 01 N 53/00, C 07 C 121/75, C 07 D 317/52

(54) **Substituierte Styrylcyclopropancarbonsäurederivate, Verfahren zu ihrer Herstellung, ihre Verwendung, insektizide und akarizide Mittel, deren Herstellung und Verfahren zur Bekämpfung von Insekten.**

(30) Priorität: **23.04.79 DE 2916358**

(43) Veröffentlichungstag der Anmeldung: **26.11.80 Patentblatt 80/24**

(45) Bekanntmachung des Hinweises auf die Patenterteilung: **18.05.83 Patentblatt 83/20**

(84) Benannte Vertragsstaaten: **AT BE CH DE FR GB IT LI NL SE**

(56) Entgegenhaltungen:
**DE-A-1 959 401**
**DE-A-1 966 839**
**DE-A-2 706 184**
**DE-A-2 738 150**
**DE-A-2 827 101**
**DE-A-2 848 495**
**FR-A-2 067 854**

(73) Patentinhaber: **BAYER AG, Zentralbereich Patente, Marken und Lizenzen, D-5090 Leverkusen 1, Bayerwerk (DE)**

(72) Erfinder: **Fuchs, Rainer, Dr., Roeberstrasse 8, D-5600 Wuppertal 1 (DE)**
Erfinder: **Hammann, Ingeborg, Dr., Belfortstrasse 9, D-5000 Köln 1 (DE)**
Erfinder: **Stendel, Wilhelm, Dr., In den Birken 55, D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 019 090 B1

Substituierte Styrylcyclopropansäurederivate, Verfahren zu ihrer Herstellung, ihre Verwendung, insektizide und akarizide Mittel, deren Herstellung und Verfahren zur Bekämpfung von Insekten.

Die Erfindung betrifft neue substituierte Styrylcyclopropancarbonsäureester, Verfahren und Zwischenprodukte zu ihrer Herstellung sowie ihre Verwendung als Insektizide und Akarizide.

Es ist bekannt, dass bestimmte Styryl-cyclopropancarbonsäure-phenoxybenzylester, wie z.B. 3-(2-Phenyl-vinyl)- und 3-(2-Chloro-2-phenylvinyl)-2,2-dimethyl-cyclopropancarbonsäure-(3-phenoxy-benzyl)-ester, insektizid und akarizid wirksam sind (vergleiche DE-OS 2 738 150). Die Wirkung dieser Verbindungen ist jedoch, insbesondere bei niedrigen Wirkstoffkonzentrationen und Aufwandmengen, nicht immer zufriedenstellend.

Es wurden gefunden:

(1) neue substituierte Styryl-cyclopropancarbonsäureester der Formel (I)

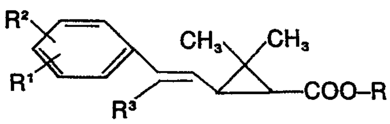
(I)

in welcher

R für den Rest eines gegebenenfalls substituierten Phenoxybenzylalkohols der allgemeinen Formel III steht

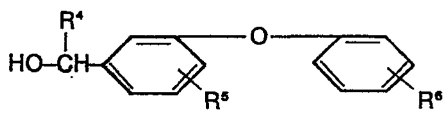
(III)

worin

$R^4$ für Wasserstoff, Cyano oder Ethinyl,

$R^5$ für Wasserstoff oder Fluor,

$R^6$ für Wasserstoff oder Fluor steht,

$R^1$ für Alkoxy oder Alkylthio, die gegebenenfalls durch Halogen substituiert sind, steht,

$R^2$ für Wasserstoff, Alkoxy oder gemeinsam mit $R^1$ für gegebenenfalls halogensubstituiertes Alkylendioxy steht, und

$R^3$ für Wasserstoff oder Chlor steht,

(2) ein Verfahren zur Herstellung von Styrylcyclopropancarbonsäureestern der Formel (I), das dadurch gekennzeichnet ist, dass man Styrylcyclopropancarbonsäuren der Formel (II)

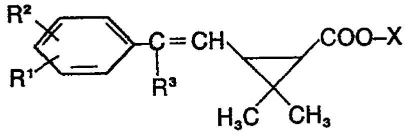
(II)

in welcher

$R^1$, $R^2$ und $R^3$ die oben angegebene Bedeutung haben, oder reaktionsfähige Derivate derselben, mit einem Alkohol der Formel (III)

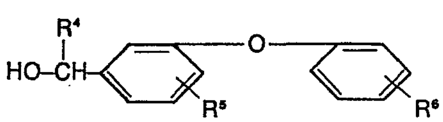
(III)

worin

$R^4$ für Wasserstoff, Cyano oder Ethinyl,

$R^5$ für Wasserstoff oder Fluor,

$R^6$ für Wasserstoff oder Fluor steht, oder dessen reaktionsfähigen Derivaten umsetzt;

(3) neue Styrylcyclopropancarbonsäuren und deren niedere Alkylester der Formel

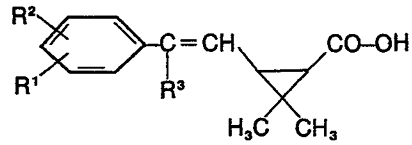
(II)

in welcher

$R^1$ für halogensubstituiertes Alkoxy steht,

$R^2$ für Wasserstoff steht,

$R^3$ für Wasserstoff oder Chlor steht und

X für Wasserstoff oder $C_{1-4}$-Alkyl steht.

Die Styrylcyclopropancarbonsäuren und deren Alkylester der Formel (II) werden hergestellt, indem man substituierte Benzylphosphonsäureester der Formel IV

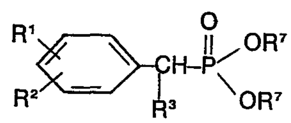
(IV)

in welcher

$R^1$, $R^2$ und $R^3$ die oben angegebene Bedeutung haben und

$R^7$ für Alkyl oder Phenyl steht oder die beiden Reste $R^7$ zusammen für Alkandiyl (Alkylen) stehen,

mit Formyl-cyclopropancarbonsäureestern der Formel V

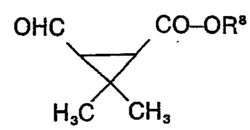

in welcher

$R^8$ für Alkyl steht, in Gegenwart von Basen und gegebenenfalls unter Verwendung von Verdünnungsmitteln umsetzt und gegebenenfalls die dabei gebildeten Styrylcyclopropancarbonsäureester nach bekannten Methoden, beispielsweise durch Erhitzen mit Natriumhydroxid in wässrigem Alkohol, zu den entsprechenden Styrylcyclopropancarbonsäuren der Formel (II) verseift.

Die allgemeine Formel (I) schliesst die verschiedenen möglichen Stereoisomeren – auch die optisch aktiven Isomeren – und deren Mischungen mit ein.

Die neuen Verbindungen der Formel (I) zeichnen sich durch hohe insektizide und akarizide Wirksamkeit aus.

Überraschenderweise zeigen die erfindungsgemässen Styrylcyclopropancarbonsäureester (I) eine erheblich höhere insektizide und akarizide Wirkung als aus dem Stand der Technik bekannte Verbindungen analoger Konstitution und gleicher Wirkungsrichtung.

Gegenstand der Erfindung sind vorzugsweise Styrylcyclopropancarbonsäurederivate der Formel (I), in welcher

$R^1$ für $C_1$–$C_4$-Alkoxy, $C_1$–$C_4$-Alkylthio, $C_1$–$C_2$-Fluoralkoxy, $C_1$–$C_2$-Chlorfluoralkoxy oder $C_1$–$C_2$-Fluoralkylthio steht,

$R^2$ für Wasserstoff, Methoxy oder zusammen mit $R^1$ für $C_1$–$C_2$-Alkylendioxy oder $C_1$–$C_2$-Fluoralkylendioxy steht,

$R^3$ für Chlor steht,

R für gegebenenfalls substituierte Phenoxybenzylalkohole der allgemeinen Formel (III)

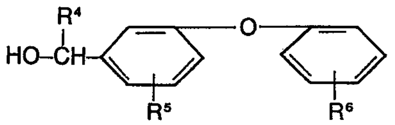

(III)

steht, worin

$R^4$ für Wasserstoff, Cyano oder Ethinyl steht und $R^5$ und $R^6$ für Wasserstoff oder Fluor stehen.

In einem bevorzugten Verfahren (a) zur Herstellung der neuen Verbindungen der Formel (I) werden als reaktionsfähige Derivate der Styrylcyclopropancarbonsäuren der Formel (II) die entsprechenden Carbonsäurechloride der Formel IIa

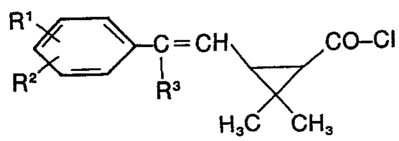

(IIa)

in welcher

$R^1$, $R^2$ und $R^3$ die oben angegebene Bedeutung haben,

mit Phenoxybenzylakoholen der Formel III

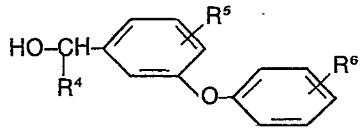

(III)

in welcher

$R^4$, $R^5$ und $R^6$ die oben angegebene Bedeutung haben, in Gegenwart eines Säureakzeptors und unter Verwendung eines Verdünnungsmittels umgesetzt.

Ein besonders bevorzugtes Verfahren (b) zur Herstellung von Verbindungen der Formel (I), worin $R^4$ für Cyano steht, ist dadurch gekennzeichnet, dass man Carbonsäurechloride der Formel IIa (oben) mit Phenoxybenzaldehyden der Formel IIIa

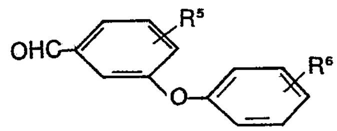

(IIIa)

in welcher

$R^4$, $R^5$ und $R^6$ die oben angegebene Bedeutung haben, in Gegenwart von wenigstens der äquimolaren Menge Alkalicyanid (z. B. Natrium- oder Kaliumcyanid) sowie in Gegenwart eines Katalysators und unter Verwendung von Verdünnungsmitteln umsetzt.

Verwendet man beispielsweise bei der bevorzugten Verfahrensvariante (a) 3-(2-Chloro-2-(4-methoxy-phenyl)-vinyl)-2,2-dimethyl-cyclopropan-1-carbonsäurechlorid und 3-(3-Fluoro-phenoxy)-benzylalkohol und bei der besonders bevorzugten Verfahrensvariante (b) 3-(2-Chloro-2-(4-trifluoromethoxy-phenyl)-vinyl)-2,2-dimethyl-cyclopropan-1-carbonsäurechlorid und 3-Phenoxy-4-fluorobenzaldehyd sowie Natriumcyanid als Ausgangsstoffe, so können die entsprechenden Reaktionen durch folgende Formelschemata skizziert werden:

(a)

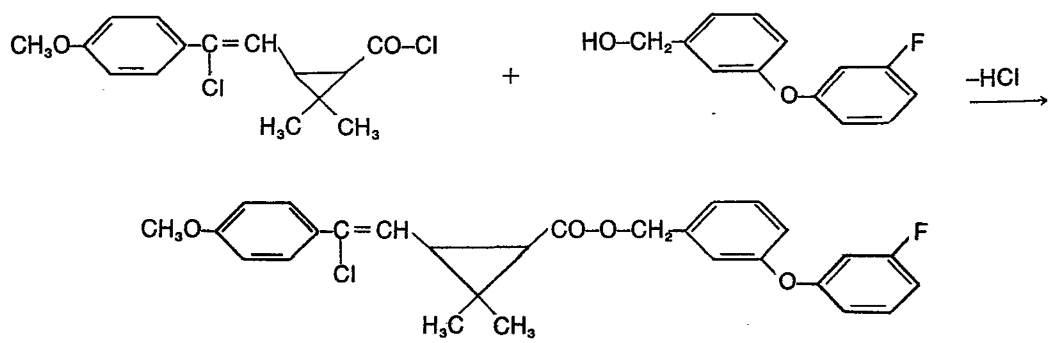


(b)

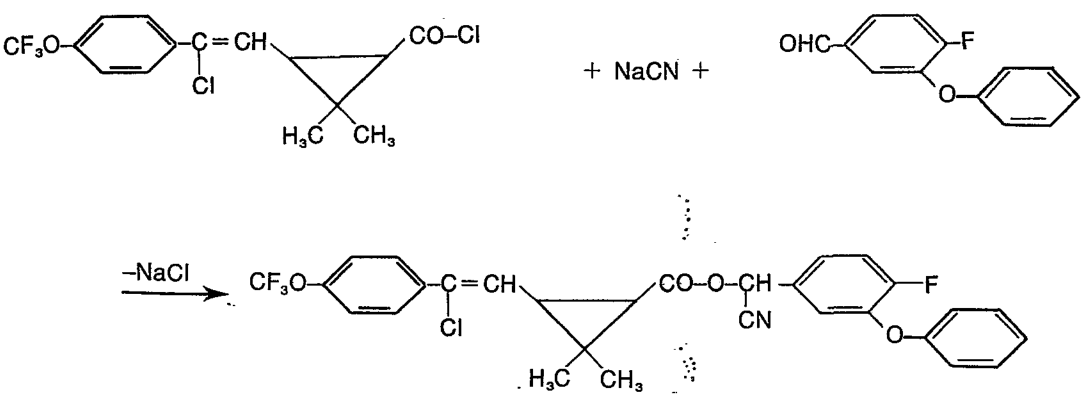

Die zu verwendenden Ausgangsstoffe sind durch die Formeln (II) bzw. (IIa) und (III) bzw. (IIIa) definiert. Vorzugsweise stehen darin die Reste R bis $R^6$ für diejenigen Reste, welche bei der Definition der Reste R bis $R^6$ in Formel (I) als bevorzugt genannt wurden.

Die als Ausgangsverbindungen zu verwendenden Styrylcyclopropancarbonsäuren (II) bzw. die entsprechenden Säurechloride (IIa) sind neu. Man erhält die Carbonsäuren der Formel (II) aus den entsprechenden Alkylestern, vorzugsweise den Methyl- oder Äthylestern, durch Verseifung, d.h. durch mehrstündiges Erhitzen auf Temperaturen zwischen 50 und 150 °C, beispielsweise mit Natriumhydroxid in wässrigem Alkohol. Die Aufarbeitung erfolgt auf übliche Weise, z.B. durch Abziehen des Alkohols im Vakuum, Verdünnen mit Methylenchlorid, Trocknen der organischen Phase und Abziehen des Lösungsmittels.

Die den Carbonsäuren der Formel (II) entsprechenden Ester erhält man, wie oben unter (4) erläutert, durch Umsetzung von substituierten Benzyl-phosphonsäureestern der Formel IV (oben) mit Formyl-cyclopropancarbonsäureestern der Formel V (oben) in Gegenwart einer Base, wie z.B. Natriummethylat, und gegebenenfalls unter Verwendung von Verdünnungsmitteln, wie z.B. Äthanol und Tetrahydrofuran, bei Temperaturen zwischen −10 und +50 °C. Die Aufarbeitung kann auf übliche Weise durchgeführt werden, z.B. indem man das Reaktionsgemisch mit Wasser verdünnt, mit Methylenchlorid extrahiert, die organische Phase trocknet, das Lösungsmittel abzieht und das zurückbleibende Produkt im Vakuum destilliert. Die den Carbonsäuren der Formel (II) entsprechenden Säurechloride der Formel (II a) können durch Umsetzung der Carbonsäuren (II) mit einem Halogenierungsmittel wie z.B. Thionylchlorid, gegebenenfalls unter Verwendung eines Verdünnungsmittels, wie z.B. Tetrachlorkohlenstoff, bei Temperaturen zwischen 10 und 100 °C hergestellt und durch Vakuumdestillation gereinigt werden.

Als Beispiele für die Carbonsäuren der Formel (II) sowie für die entsprechenden Säurechloride (IIa) und Ester seien genannt:

3-(2-Chloro-2-(4-methoxy-phenyl)-vinyl-,
3-(2-Chloro-2-(4-äthoxy-phenyl)-vinyl-,
3-(2-Chloro-2-(3,4-dimethoxy-phenyl)-vinyl-,
3-(2-Chloro-2-(4-methylthio-phenyl)-vinyl-,
3-(2-Chloro-2-(3,4-methylendioxy-phenyl)-vinyl-,
3-(2-Chloro-2-(4-trifluormethoxy-phenyl)-vinyl-,
3-(2-Chloro-2-(4-chlordifluormethoxy-phenyl)-vinyl-,
3-(2-Chloro-2-(4-(1,1,2,2-tetrafluoräthoxy-phenyl)-vinyl-,
3-(2-Chloro-2-(4-(2-chlor-1,1,2-trifluor-äthoxy)-phenyl)-vinyl-,
3-(2-Chloro-2-(4-trifluormethylthio-phenyl)-vinyl-,
3-(2-Chloro-2-(3,4-difluormethylendioxy-phenyl)-vinyl-
und
3-(2-Chloro-2-(3,4-trifluoräthylendioxy-phenyl)-vinyl-2,2-dimethyl-cyclopropancarbonsäure sowie die entsprechenden Säurechloride, die Methylester und die Äthylester.

Die als Zwischenprodukte zu verwendenden substituierten Benzylphosphonsäureester sind durch Formel (IV) definiert. Vorzugsweise stehen darin

$R^1$, $R^2$ und $R^3$ für diejenigen Reste, welche bei der Definition der Reste $R^1$, $R^2$ und $R^3$ in Formel (I) als bevorzugt genannt sind, und

$R^7$ steht vorzugsweise für Methyl, Äthyl, Phenyl, oder beide Reste $R^7$ stehen zusammen für 2,2-Dimethyl-propan-1,3-diyl.

Als Beispiele seien genannt:
4-Methoxy-, 4-Äthoxy-, 3,4-Dimethoxy-, 4-Methylthio-, 3,4-Methylendioxy-, 4-Trifluormethoxy-, 4-Chlordifluormethoxy-, 4-(1,1,2,2-Tetrafluoräthoxy)-, 4-(2-Chlor-1,1,2-trifluor-äthoxy)-, 4-Trifluormethylthio-, 3,4-Difluormethylendioxy- und 3,4-Trifluoräthylendioxy-α-chloro-benzyl-phosphonsäuredimethylester, -α-chloro-benzyl-phosphonsäurediäthylester und α-chloro-benzyl-phosphonsäure-diphenylester.

Die in α-Stellung unsubstituierten Benzylphosphonsäureester der Formel (IV) sind bekannt bzw. lassen sich nach bekannten Verfahren herstellen.

Die in α-Stellung durch Cl substituierten Benzylphosphonsäureester der Formel (IV) sind nicht in der Literatur beschrieben. Man erhält sie durch Umsetzung von α-Hydroxy-benzylphosphonsäureestern der Formel

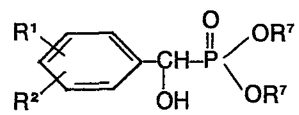

(VI)

in welcher

$R^1$, $R^2$ und $R^7$ die oben angegebene Bedeutung haben, mit Chlorierungsmitteln wie z.B. Thionylchlorid bei Temperaturen zwischen 0 und 100 °C, gegebenenfalls in Gegenwart eines basischen Katalysators wie z.B. Pyridin und gegebenenfalls unter Verwendung eines Verdünnungsmittels wie z.B. Methylenchlorid (vergleiche z.B. Chimia 28 (1974), 656–657; J.Am.Chem.Soc. 87 (1965), 2777–2778).

Die α-Hydroxy-benzylphosphonester, welche als Zwischenprodukte verwendet werden können, sind durch Formel (VI) definiert. Vorzugsweise stehen darin

$R^1$, $R^2$ und $R^7$ für diejenigen Reste, welche bei der Definition der Reste $R^1$ und $R^2$ in Formel (I) bzw. von $R^7$ in Formel (IV) als bevorzugt genannt sind.

Als Beispiele seien genannt:
4-Methoxy-, 4-Äthoxy-, 3,4-Dimethoxy-, 4-Methylthio-, 3,4-Methylendioxy-, 4-Trifluormethoxy-, 4-Chlordifluormethoxy-, 4-(1,1,2,2,-Tetrafluoräthoxy)-, 4-(2-Chlor-1,1,2-trifluor-äthoxy)-, 4-Trifluormethylthio-, 3,4-Difluormethylendioxy- und 3,4-Trifluoräthylendioxy-α-hydroxy-benzyl-phosphonsäuredimethylester, -α-hydroxy-benzylphosphonsäure-diäthylester und -α-hydroxy-benzylphosphonsäure-diphenylester.

Die α-Hydroxy-benzylphosphonsäureester der Formel (VI) sind neue Verbindungen. Man erhält

sie nach im Prinzip bekannten Verfahren, im allgemeinen durch Umsetzung von Aldehyden der Formel VII

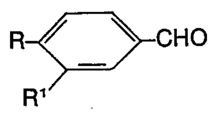 (VII)

in welcher

R und $R^1$ die oben angegebene Bedeutung haben, mit Phosphorigsäureestern der Formel

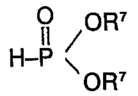 (VIII)

worin $R^7$ die oben angegebene Bedeutung hat, gegebenenfalls in Gegenwart eines Katalysators wie z. B. Triäthylamin, bei Temperaturen zwischen 0 und 150 °C, vorzugsweise bei 20 bis 100 °C (vergleiche Houben-Weyl, Methoden der organischen Chemie, 4. Auflage (1963), Band 12/1, S. 475–483, Georg Thieme Verlag, Stuttgart).

Als Beispiele für die Aldehyde der Formel (VII) seien genannt:

4-Methoxy-, 4-Äthoxy-, 3,4-Dimethoxy-, 4-Methylthio-, 3,4-Methylendioxy-, 4-Trifluormethoxy-, 4-Chlordifluormethoxy-, 4-(1,1,2,2-Tetrafluor-äthoxy)-, 4-(2-Chlor-1,1,2-trifluor-äthoxy)-, 4-Trifluormethylthio-, 3,4-Difluormethylendioxy- und 3,4-Trifluoräthylendioxybenzaldehyd.

Aldehyde der Formel (VII) sind bekannt (vergleiche z. B. J. Gen. Chem. USSR 30 (1960), 3103; Bull. Soc. Chim. France 1955, 1594 ibid. 1962, 254–262; J. Org. Che. 37 (1972), 673; DE-OS 2 029 556; US-PS 3 387 037; J. Med. Chem. 16 (1973), 1399).

Als Beispiele für die Phosphorigsäureester der Formel (VIII) seien genannt:
Phosphorigsäure-dimethylester (Dimethylphosphit), Phosphorigsäure-diäthylester (Diäthylphosphit) und Phosphorigsäure-diphenylester (Diphenylphosphit).

Die Phosphorigsäureester der Formel (VIII) sind bekannte Verbindungen.

Die weiter als Zwischenprodukte zu verwendenden Formylcyclopropan-carbonsäureester sind durch Formel (V) definiert. Vorzugsweise steht darin

$R^8$ für geradkettiges oder verzweigtes Alkyl mit 1 bis 4, insbesondere 1 oder 2 Kohlenstoffatomen.

Als Beispiele für die Verbindungen der Formel (V) seien im einzelnen genannt:

2,2-Dimethyl-3-formyl-cyclopropan-1-carbonsäure-methylester und -äthylester.

Die Verbindungen der Formel (V) sind bekannt oder können nach bekannten Verfahren, im allgemeinen durch Umsetzung von bekannten Alken(1)yl-cyclopropancarbonsäureestern mit Ozon hergestellt werden (vergleiche z. B. US-PS 3 679 667).

Bevorzugte Alkohole zur Herstellung der Verbindungen der Formel (I) sind:

3-Phenoxy-, 3-(3-Fluoro-phenoxy)-, 3-(4-Fluorophenoxy)-, 3-Phenoxy-4-fluoro-, 3-(3-Fluoro-phenoxy)-4-fluoro- und 3-(4-Fluoro-phenoxy)-4-fluorobenzylalkohol, 3-(Phenoxy-, 3-(3-Fluoro-phenoxy)-, 3-(4-Fluoro-phenoxy)-, 3-Phenoxy-4-fluoro-, 3-(3-Fluoro-phenoxy)-4-fluoro- und 3-(4-Fluoro-phenoxy)-4-fluoro-α-cyano-benzylalkohol, 3-Phenoxy-, 3-(3-Fluoro-phenoxy)-, 3-(4-Fluoro-phenoxy)-, 3-Phenoxy-4-fluoro-, 3-(3-Fluoro-phenoxy)-4-fluoro- und 3-(4-Fluoro-phenoxy)-4-fluoro-α-äthinyl-benzylalkohol sowie 3-Phenoxy-, 3-(3-Fluoro-phenoxy)-, 3-(4-Fluoro-phenoxy)-, 3-Phenoxy-4-fluoro-, 3-(3-Fluoro-phenoxy)-4-fluoro- und 3-(4-Fluorophenoxy)-4-fluoro-benzaldehyd.

Das Verfahren zur Herstellung der erfindungsgemässen Styryl-cyclopropancarbonsäureester wird bevorzugt unter Verwendung geeigneter Lösungs- oder Verdünnungsmittel durchgeführt. Als solche kommen praktisch alle inerten organischen Solventien in Frage. Hierzu gehören insbesondere aliphatische und aromatische, gegebenenfalls chlorierte Kohlenwasserstoffe, wie Benzin, Benzol, Toluol, Xylol, Methylenchlorid, Chloroform, Tetrachlorkohlenstoff, Chlorbenzol und o-Dichlorbenzol, Äther wie Diäthyl- und Dibutyläther, Tetrahydrofuran und Dioxan, Ketone wie Aceton, Methyläthyl-, Methylisopropyl- und Methylisobutylketon sowie Nitrile wie Acetonitril und Propionitril.

Beim Arbeiten im Zweiphasenmedium wird Wasser als zweite Lösungsmittelkomponente verwendet. Bei dem als bevorzugt beschriebenen Verfahren, ausgehend von Säurechloriden der Formel (IIa) und Phenoxybenzylalkoholen der Formel (III), können als Säureakzeptoren alle üblichen Säurebindemittel Verwendung finden. Besonders bewährt haben sich Alkalicarbonate und -alkoholate, wie Natrium- und Kaliumcarbonat, Natrium- und Kaliummethylat bzw. -äthylat, ferner aliphatische, aromatische oder heterocyclische Amine, beispielsweise Triäthylamin, Trimethylamin, Dimethylanilin, Dimethylbenzylamin und Pyridin.

Bei dem als besonders bevorzugt beschriebenen Verfahren, ausgehend von Säurechloriden der Formel (IIa) und Phenoxybenzaldehyden der Formel (IIIa), werden als Katalysatoren im allgemeinen Verbindungen verwendet, welche gewöhnlich bei Reaktionen in mehrphasigen Medien als Hilfsmittel zum Phasentransfer von Reaktanden dienen. Insbesondere seien Tetraalkyl- und Trialkyl-aralkyl-ammoniumsalze wie z. B. Tetrabutylammoniumbromid und Trimethyl-benzylammoniumchlorid genannt.

Die Reaktionstemperatur kann innerhalb eines grösseren Bereichs variiert werden. Im allgemeinen arbeitet man zwischen 0 und 100 °C, vorzugsweise bei 10 bis 50 °C. Das erfindungsgemässe Verfahren wird im allgemeinen bei Normaldruck durchgeführt.

Zur Durchführung des erfindungsgemässen Verfahrens werden die Ausgangsstoffe gewöhnlich in äquimolaren Mengen eingesetzt. Ein Überschuss der einen oder anderen Reaktionskomponente bringt keine wesentlichen Vorteile. Die Umsetzung wird im allgemeinen in einem oder mehreren Verdünnungsmitteln in Gegenwart eines Säureakzeptors oder eines Katalysators durchgeführt, und das Reaktionsgemisch wird mehrere Stunden bei der erforderlichen Temperatur gerührt. Anschliessend wird das Reaktionsgemisch mit Toluol/Wasser geschüttelt, die organische Phase abgetrennt, mit Wasser gewaschen und getrocknet. Nach Abdestillieren des Lösungsmittels im Vakuum fallen im allgemeinen die neuen Verbindungen in Form von Ölen an, die sich zum Teil nicht unzersetzt destillieren lassen, jedoch durch sogenanntes «Andestillieren», d.h. durch längeres Erhitzen unter vermindertem Druck auf mässig erhöhte Temperaturen von den letzten flüchtigen Anteilen befreit und auf diese Weise gereinigt werden. Zu ihrer Charakterisierung dient der Brechungsindex.

Die neuen Styryl-cyclopropancarbonsäureester zeichnen sich, wie bereits erwähnt, durch eine hohe insektizide und akarizide Wirksamkeit aus.

Die Wirkstoffe eignen sich bei guter Pflanzenverträglichkeit und günstiger Warmblütertoxizität zur Bekämpfung von tierischen Schädlingen, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber. Aus der Ordnung der Diplopoda z.B. Blaniulus guttulatus. Aus der Ordnung der Chilopoda z.B. Geophilus carpophagus, Scutigera spec. Aus der Ordnung der Symphyla z.B. Scutigerella immaculata. Aus der Ordnung der Thysanura z.B. Lepisma saccharina. Aus der Ordnung der Collembola z.B. Onychiurus armatus. Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria. Aus der Ordnung der Dermaptera z.B. Forficula auricularia. Aus der Ordnung der Isoptera z.B. Reticulitermes spp. Aus der Ordnung der Anoplura z.B. Phylloxera vastatrix, Pemphigus spp., Pediculus humanus corporis, Haematopinus spp., Linognathus spp. Aus der Ordnung der Mallophaga z.B. Trichodectes spp., Damalinea spp. Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp. Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp. Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia amibguella, Homona magnanima, Tortrix viridana. Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica. Aus der Ordnung der Hymenoptera z.B. Diprion spp., Hoplocampa spp., Lasius spp., Monomorium pharaonis, Vespa spp. Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp. Aus der Ordnung der Arachnida z.B. Scorpio maurus, Latrodectus mactans. Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykoläther, Alkylsulfonate, Alkylsulfate, Arylsulfonate sowie Eiweisshydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die Anwendung der erfindungsgemässen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,01 und 10 Gew.-% liegen.

Die Anwendung geschieht in einer den Anwendungsformen angepassten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemässen Wirkstoffe eignen sich auch zur Bekämpfung von Ekto- und Endoparasiten auf dem veterinärmedizinischen Gebiet.

Die Anwendung der erfindungsgemässen Wirkstoffe geschieht im Veterinärsektor in bekannter Weise, wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgiessens (pour-on and spot-on) und des Einpuderns sowie durch parenterale Anwendung in Form beispielsweise der Injektion.

Beispiel A
Phaedon-Larven-Test

Lösungsmittel: 3 Gewichtsteile Aceton
Emulgator: 1 Gewichtsteil Alkylarylpolyglykoläther

Zur Herstellung einer zweckmässigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100%, dass alle Käfer-Larven abgetötet wurden; 0% bedeutet, dass keine Käfer-Larven abgetötet wurden.

Bei diesem Test zeigen z.B. die folgenden Verbindungen der Herstellungsbeispiele überlegene Wirksamkeit gegenüber dem Stand der Technik: 1, 2, 6, 8.

Beispiel B
Tetranychus-Test (resistent)
Lösungsmittel: 3 Gewichtsteile Aceton
Emulgator: 1 Gewichtsteil Alkylarylpolyglykoläther

Zur Herstellung einer zweckmässigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100%, dass alle Spinnmilben abgetötet wurden; 0% bedeutet, dass keine Spinnmilben abgetötet wurden.

Bei diesem Test zeigen z.B. die folgenden Verbindungen der Herstellungsbeispiele überlegene Wirksamkeit gegenüber dem Stand der Technik: 7, 1, 2.

Beispiel C
Test mit Boophilus microplus resistent
Lösungsmittel:
35 Gewichtsteile Äthylenglykolmonomethyläther
35 Gewichtsteile Nonylphenolpolyglykoläther

Zur Herstellung einer zweckmässigen Wirkstoffzubereitung vermischt man drei Gewichtsteile Wirkstoff mit sieben Gewichtsteilen des oben angegebenen Lösungsmittel-Gemisches und verdünnt das so erhaltene Konzentrat mit Wasser auf die gewünschte Konzentration.

10 adulte Boophilus microplus res. werden in die zu testende Wirkstoffzubereitung 1 Minute getaucht. Nach Überführung in Plastikbecher und Aufbewahrung in einem klimatisierten Raum wird der Abtötungsgrad bestimmt.

Bei diesem Test zeigen z.B. die folgenden Verbindungen der Herstellungsbeispiele überlegene Wirkung gegenüber dem Stand der Technik: 7, 3, 2, 1, 4, 8.

Herstellungsbeispiele
Beispiel 1

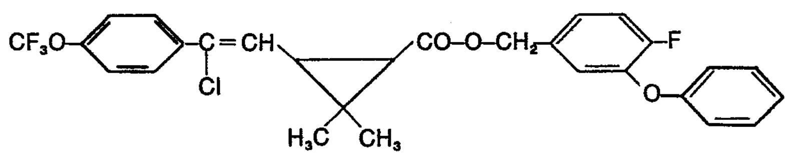

4,4 g (0,02 Mol) 3-Phenoxy-4-fluoro-benzylalkohol und 7,1 g (0,02 Mol) 2,2-Dimethyl-3-(2-chloro-2(4-trifluormethoxy-phenyl)-vinyl)-cyclopropancarbonsäurechlorid werden in 100 ml wasserfreiem Toluol gelöst und bei 20–25 °C 2,5 g Pyridin, gelöst in 50 ml wasserfreiem Toluol, unter Rühren zugetropft. Anschliessend wird weitere 3 Stunden bei 25–35 °C gerührt. Das Reaktionsgemisch wird in 150 ml Wasser, dem 10 ml konzentrierte Salzsäure zugesetzt werden, gegossen, die organische Phase abgetrennt und nochmals mit 100 ml Wasser gewaschen. Anschliessend wird die Toluolphase über Natriumsulfat getrocknet und das Lösungsmittel im Wasserstrahlvakuum abdestilliert. Letzte Lösungsmittelreste werden durch kurzes Andestillieren bei 60 °C/l Torr Badtemperatur entfernt. Man erhält 8,7 g (81,4% der Theorie) 2,2-Dimethyl-3-(2-chloro-2-(4-trifluormethoxy-phenyl)-vinyl)-cyclopropancarbonsäure-(4-fluoro-3-phenoxy-benzyl)-ester als gelbes Öl mit dem Brechungsindex $n_D^{23}$ : 1,5443.

Beispiel 2

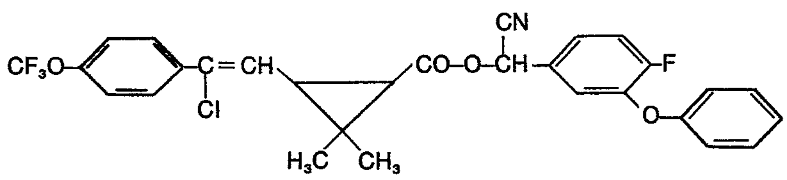

4,32 g (0,02 Mol) 3-Phenoxy-4-fluoro-benzaldehyd und 7,1 g (0,02 Mol) 2,2-Dimethyl-3-(2-chloro-2-(4-trifluormethoxy-phenyl)-vinyl)-cyclopropancarbonsäurechlorid werden zusammen unter Rühren bei 20–25 °C zu einer Mischung von 1,6 g Natriumcyanid, 2,5 ml Wasser, 100 ml n-Hexan und 0,5 g Tetrabutylammoniumbromid getropft und dann 4 Stunden bei 20–25 °C gerührt. Anschliessend wird die Reaktionsmischung mit 300 ml Toluol versetzt und 2mal mit je 300 ml Wasser ausgeschüttelt. Die organische Phase wird abgetrennt, über Magnesiumsulfat getrocknet und das Lösungsmittel im Wasserstrahlvakuum abdestilliert. Letzte Lösungsmittelreste werden durch kurzes Andestil-

lieren bei 60 °C/l Torr Badtemperatur entfernt. Man erhält 7 g (62,6% der Theorie) 2,2-Dimethyl-3-(2-chloro-2-(4-trifluormethoxy-phenyl)-vinyl)-cyclopropancarbonsäure-3-phenoxy-4-fluoro-α-cyano-benzylester als zähes Öl. Die Struktur wird durch das $^1$H-NMR-Spektrum bestätigt.

$^1$H-NMR-Spektrum ($CDCl_3$/TMS):
aromatische-H: 7,74–6,76 δ (m/12 H), benzyl-H: 6,48–6,27 δ (m/1 H), vinyl-H: 6,0–5,62 δ (m/1 H) cyclopropan-H: 2,75–1,55 δ (m/2 H), dimethyl-H: 1,5–1,12 δ (m/6 H).

Analog Beispiel 1 und/oder 2 erhält man:

(3)

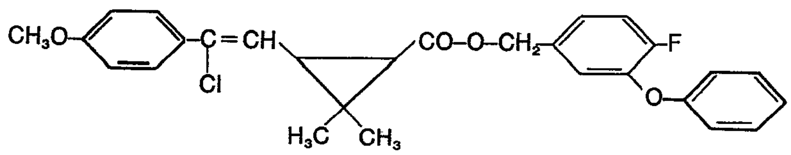

Ausbeute: 84,5% der Theorie

(4)

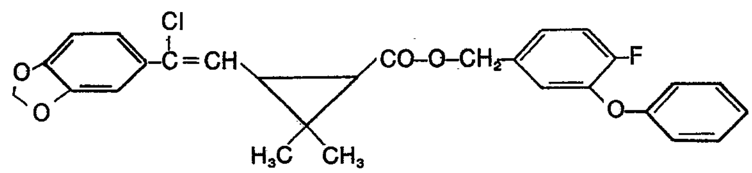

Ausbeute: 82,9% der Theorie

$^1$H-NMR-Spektrum ($CDCl_3$/TMS):
aromatische-H: 7,65–6,7 δ (m/11 H), methylendioxy-H: 5,96 δ (S/2H), vinyl-H: 5,8–5,5 δ (m/1 H), benzyl-H: 5,14–5,07 δ (m/2 H), cyclopropan-H: 2,7–1,45 δ (m/2 H), dimethyl-H: 2,5–1,0 δ (m/6 H).

(5)

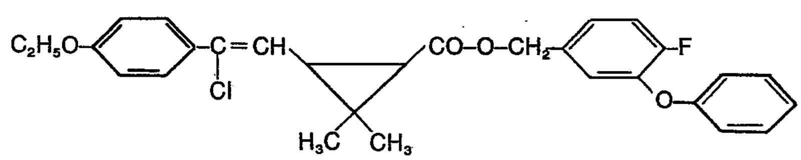

Ausbeute: 79,3% der Theorie

(6)

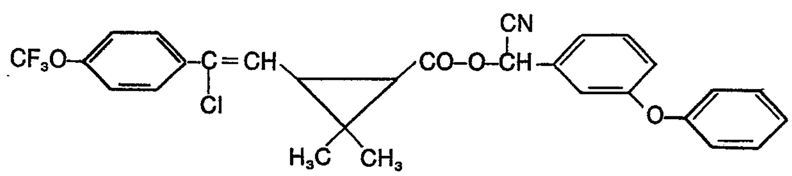

Ausbeute: 72,5% der Theorie

(7)

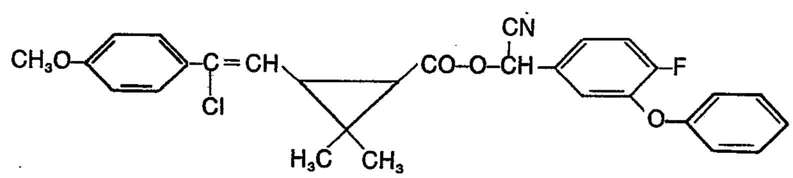

Ausbeute: 74,6% der Theorie

$^1$H-NMR-Spektrum ($CDCl_3$/TMS):
aromatische-H: 7,6–6,7 δ (m/12 H), benzyl-H: 6,4–6,22 δ (m/1 H), vinyl-H: 5,83–5,5 δ (m/1 H), methoxy-H: 3,78 δ (S/3 H), cyclopropan-H: 2,7–1,45 δ (m /2 H), dimethyl-H: 1,4–1,1 δ (m/6 H).

(8)

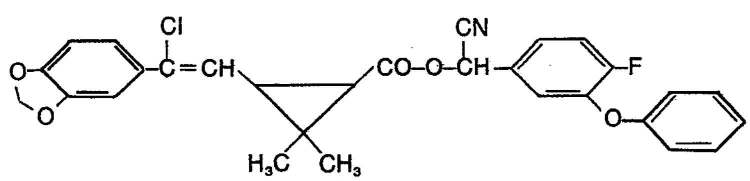

Ausbeute: 67,3% der Theorie

$^1$H-NMR-Spektrum ($CDCl_3$/TMS)
aromatische-H: 7,67–6,7 δ (m11 H), benzyl-H: 6,43–6,24 δ (m/1 H), methylendioxy-H: 5,95 δ (S/2 H), vinyl-H: 5,8–5,5 δ (m/1 H), cyclopropan-H: 2,74–1,48 δ (m/2 H), dimethyl-H:1,4–0,9 δ (m/6 H).

(9)

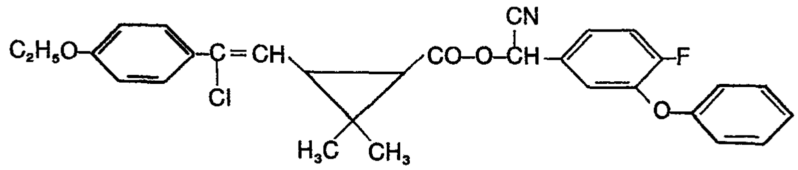

Ausbeute: 72,8% der Theorie

(10)

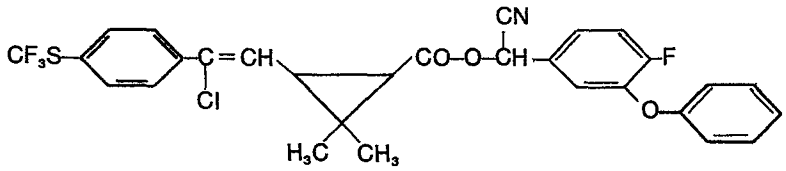

(11)

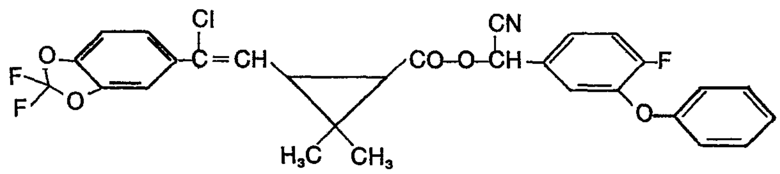

(12)

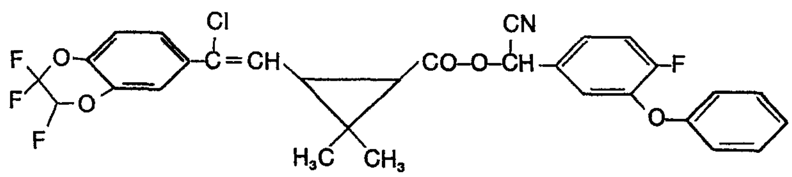

(13)

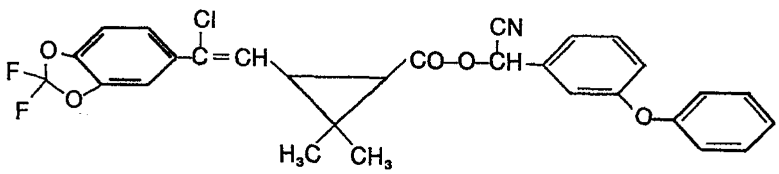

Die Ausgangsverbindungen können wie folgt hergestellt werden:

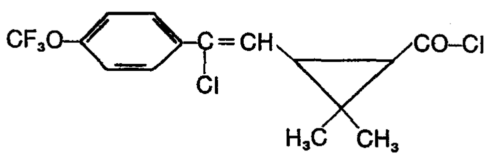

19,4 g (0,058 Mol) 2,2-Dimethyl-3-(2-chloro-2-(4-trifluormethoxy-phenyl)-vinyl)-cyclopropancarbonsäure werden in 150 ml Tetrachlorkohlenstoff gelöst und bei 25 °C langsam unter Rühren 15 g Thionylchlorid zugetropft. Anschliessend wird 4 Stunden zum Rückfluss erhitzt. Nach beendeter Reaktionszeit werden überschüssiges Thionylchlorid sowie Tetrachlorkohlenstoff im Wasserstrahlvakuum abdestilliert. Letzte Lösungsmittelreste werden durch kurzes Andestillieren bei 2 Torr/60 °C Badtemperatur entfernt. Man erhält 19,4 g (95% der Theorie) 2,2-Dimethyl-3-(2-chloro-2-(4-trifluormethoxy-phenyl)-vinyl)-cyclopropancarbonsäurechlorid als ölige Flüssigkeit.

Analog erhält man:

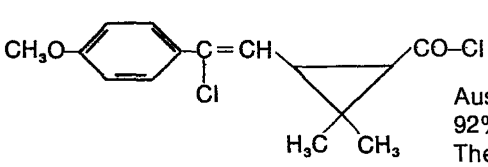

Ausbeute: 92% der Theorie

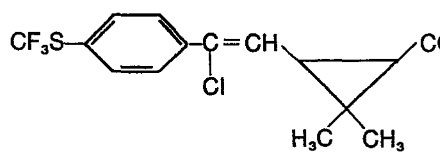

Ausbeute: 82% der Theorie

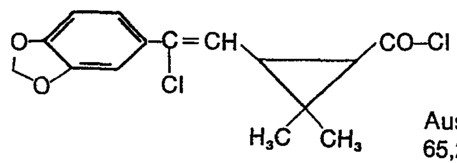

Ausbeute: 65,2% der Theorie

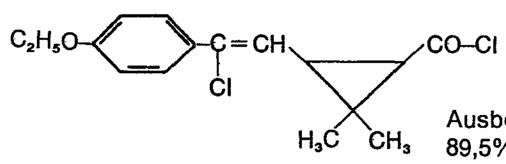

Ausbeute: 89,5% der Theorie

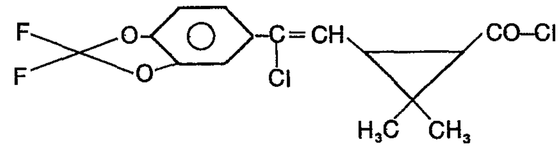

Ausbeute: 85,2% der Theorie

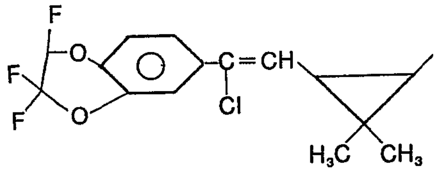

Ausbeute: 82,3% der Theorie

Herstellung von

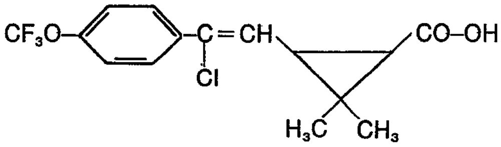

30,8 g (0,085 Mol) 2,2-Dimethyl-3-(2-chloro-2-(4-trifluormethoxy-phenyl)-vinyl)-cyclopropancarbonsäureäthylester werden in 100 ml Äthanol gelöst, dann mit einer Lösung von 4 g (0,1 Mol) Natriumhydroxid in 100 ml Wasser versetzt und 4 Stunden unter Rühren zum Rückfluss erhitzt. Anschliessend wird das Äthanol im Wasserstrahlvakuum abdestilliert, der Rückstand in 300 ml warmem Wasser aufgenommen und 1 mal mit 300 ml Methylenchlorid extrahiert. Die wässrige Phase wird abgetrennt, mit konzentrierter Salzsäure angesäuert und anschliessend mit 2mal 300 ml Methylenchlorid extrahiert. Die organische Phase wird dann abgetrennt über Magnesiumsulfat, getrocknet und das Lösungsmittel im Wasserstrahlvakuum abdestilliert. Letzte Lösungsmittelreste werden durch kurzes Andestillieren bei 2 Torr/60 °C Badtemperatur entfernt. Man erhält dann 19,4 g (68,3% der Theorie) 2,2-Dimethyl-3-(2-chloro-2-(4-trifluormethoxy-phenyl)-vinyl)-cyclopropancarbonsäure (cis und trans, E und Z-Isomerengemisch) als sehr zähes gelbes Öl. Die Struktur wird durch das $^1H$-NMR-Spektrum bestätigt.

$^1H$-NMR (in $CDCl_3$/TMS):

aromatische-H: 7,65–6,9 δ (m/4 H), vinyl-H: 5,9–5,6 δ (m/1 H), cyclopropan-H: 2,6–1,4 δ (m/2 H) und dimethyl-H: 1,4–1,0 δ (m/6 H).

Analog erhält man:

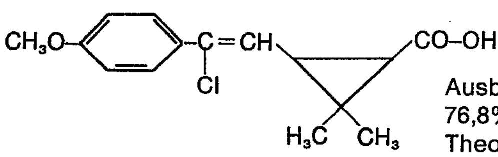

Ausbeute: 76,8% der Theorie

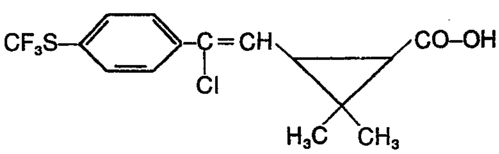

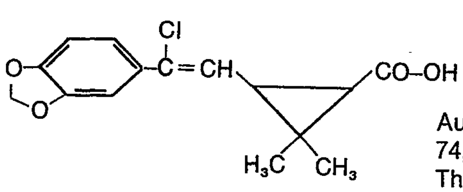

Ausbeute: 74,4% der Theorie

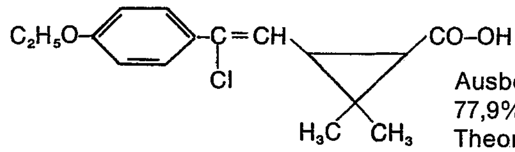

Ausbeute: 77,9% der Theorie

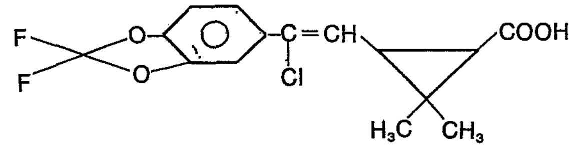

Ausbeute: 71,2% der Theorie

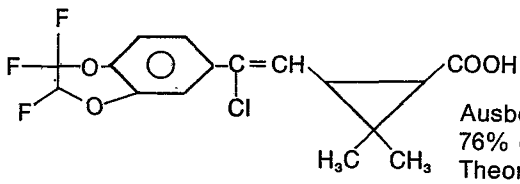

Ausbeute: 76% der Theorie

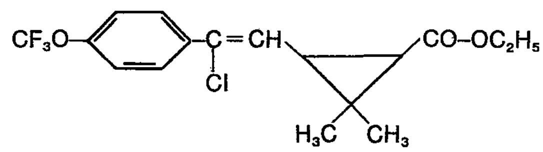

4,6 g (0,2 Mol) Natrium werden portionsweise in 100 ml Äthanol gelöst. Wenn sich alles Natrium aufgelöst hat, werden 100 ml Tetrahydrofuran (wasserfrei) zugesetzt und bei 0 °C unter Rühren 69,3 g (0,2 Mol) 4-Trifluormethoxy-α-chloro-benzyl-phosphonsäurediäthylester, gelöst in 50 ml wasserfreiem Tetrahydrofuran, zugetropft. Nachdem noch 2 Stunden bei 0–5 °C nachgerührt wurde, werden 34 g (0,2 Mol) cis/trans-2,2-Dimethyl-3-formyl-cyclopropancarbonsäureäthylester, gelöst in 50 ml wasserfreiem Tetrahydrofuran, unter Rühren bei 0 °C zugetropft. Anschliessend wird noch 12 Stunden bei 20–25 °C nachgerührt. Die Reaktionsmischung wird dann mit 600 ml Wasser versetzt und 2mal mit je 300 ml Methylenchlorid extrahiert. Die organische Phase wird abgetrennt, über Magnesiumsulfat getrocknet, das Lösungsmittel im Wasserstrahlvakuum abdestilliert und der Rückstand im Vakuum destilliert. Man erhält 42,3 g (58,3% der Theorie) 2,2-Dimethyl-3-(2-chloro-2-(4-trifluormethoxy-phenyl)-vinyl)-cyclopropancarbonsäureäthylester (cis, trans und E, Z-Isomerengemisch) als gelbes Öl mit dem Siedepunkt 160–172 °C/2 Torr.

Analog erhält man:

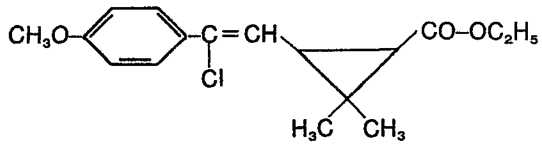

Ausbeute: 64,8% der Theorie

Siedepunkt: 160–165 °C/1 Torr

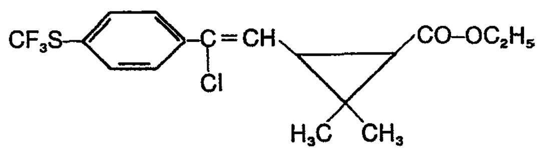

Ausbeute:
38,8% der Theorie
Siedepunkt:
160–170 °C/1 Torr

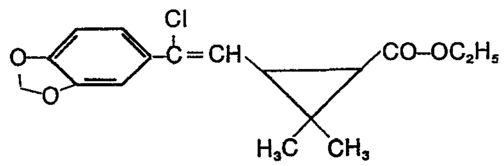

Ausbeute:
69,4% der Theorie
Siedepunkt:
201–204 °C/2 Torr

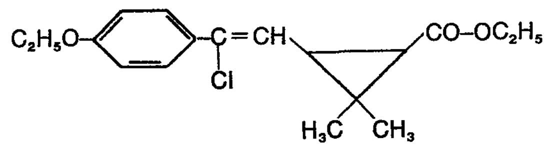

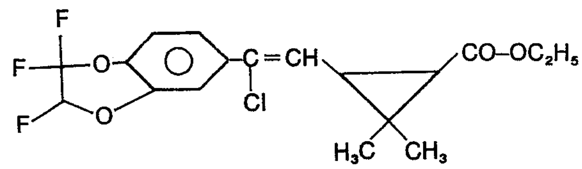

Ausbeute:
32% der Theorie
Siedepunkt:
145–155 °C/2 mm Hg

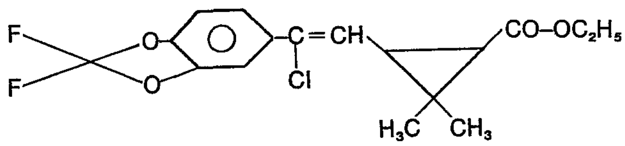

Ausbeute:
40,2% der Theorie
Siedepunkt:
155–175 °C/2 mm Hg

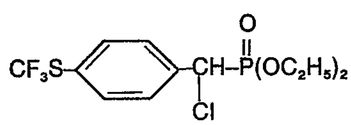

Einem Gemisch aus 22,96 g (0,07 Mol) 4-Trifluormethoxy-α-hydroxy-benzylphosphonsäure-diäthylester, 70 ml Methylenchlorid und 5,6 g (0,07 Mol) Pyridin werden bei 20–40 °C unter leichter Wasserkühlung innerhalb von etwa 1 Stunde 8,7 g (0,073 Mol) Thionylchlorid zugesetzt. Das Reaktionsgemisch wird dann 3 Stunden auf 50 °C erhitzt und 12 Stunden ohne weitere Wärmeeinwirkung nachgerührt. Das Gemisch wird auf etwa 100 g Eiswasser gegossen, die organische Phase abgetrennt und getrocknet. Nach Abdestillieren des Lösungsmittels wird der Rückstand bei 2 Torr und 45 °C eingeengt. Man erhält 18,7 g (77,1% der Theorie) 4-Trifluormethoxy-α-chloro-benzyl-phosphonsäure-diäthylester als gelbes viskoses Öl mit einem Brechungsindex von $n_D^{22}$ : 1,4968.

Analog erhält man die folgenden Verbindungen:

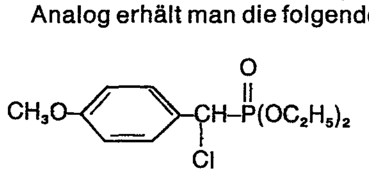

Ausbeute:
91,2% der Theorie
Brechungsindex:
$n_D^{22}$ : 1,5188

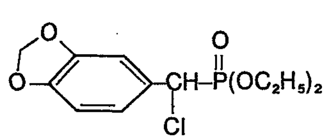

Ausbeute:
75,2% der Theorie

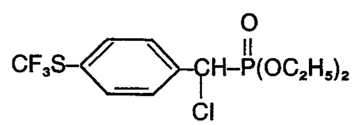

Ausbeute:
66% der Theorie

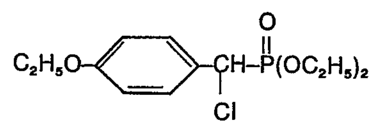

Ausbeute:
88,7% der Theorie

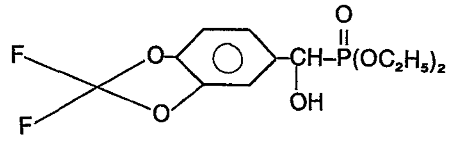

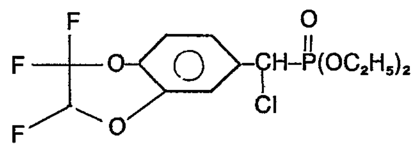

Ausbeute:
56,6% der Theorie

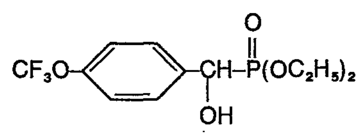

in ein Gemisch aus 13,8 g (0,1 Mol) Diäthylphosphit, 1 g Triäthylamin und 1 ml 20%iger Natriummethylatlösung werden innerhalb einer Stunde unter Wasserkühlung bei 50–70 °C 19 g (0,1 Mol)

4-(Trifluormethoxy-benzaldehyd eingeleitet. Der Reaktionsansatz wird 3 Stunden bei 60 °C nachgerührt. Nach dem Abkühlen wird der Ansatz mit 50 ml Methylenchlorid aufgenommen und mehrfach mit verdünnter Salzsäure und kaltem Wasser nachgewaschen. Die organische Schicht wird abgetrennt und im Vakuum vom Lösungsmittel befreit. Man erhält 26,2 g (79,9% der Theorie) 4-Trifluormethoxy-α-hydroxy-benzyl-phosphonsäurediäthylester als gelbes sehr viskoses Öl mit dem Brechungsindex von $n_D^{22}$:1,4582.

Analog können hergestellt werden:

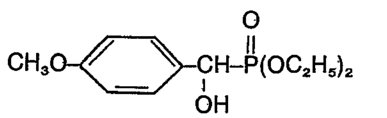
Ausbeute: 81,2% der Theorie

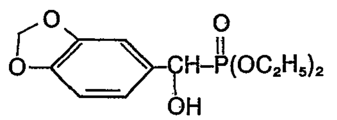
Ausbeute: 85,5% der Theorie

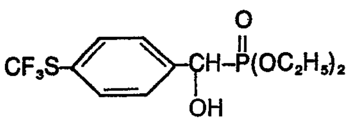
Ausbeute: 72,2% der Theorie

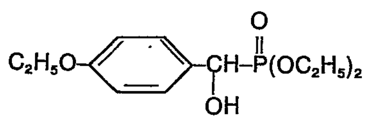
Ausbeute: 83,6% der Theorie

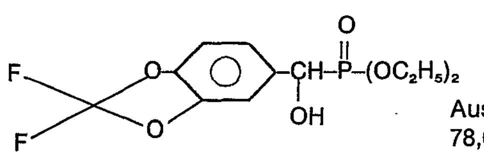
Ausbeute: 78,6% der Theorie

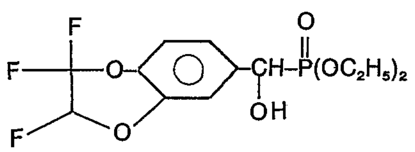
Ausbeute: 74,5% der Theorie

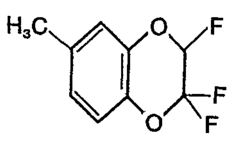

124 g 4-Methylbrenzkatechin werden zusammen mit 110 g Kaliumhydroxid in 300 ml Tetramethylensulfon bei 110 °C vorgelegt. Es werden dann innerhalb 4 Stunden 170 g Trifluorchloräthylen eingeleitet. Der Ansatz wird anschliessend bei 15 mm über eine Kolonne destilliert, wobei bis zu einer Übergangstemperatur von 85 °C abgenommen wurde. Nach Abtrennen der wässrigen Phase in der Vorlage wird das Produkt erneut destilliert. Man erhält bei einem Siedepunkt $K_{p\ 12}$: 70–72 °C ($n_D^{20}$:1,4565) 133 g (65% der Theorie) 6-Methyl-2,2,3-trifluor-1,4-benzodioxen.

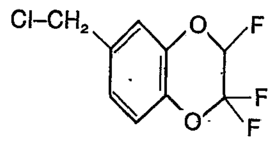
+
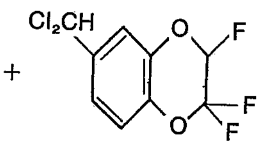

6-Chlormethyl-trifluorbenzodioxen und 6-Dichlormethyl-trifluorbenzodioxen

1065 g 6-Methyl-trifluorbenzodioxen werden unter UV-Bestrahlung bei 120 °C vorgelegt und 440 g Chlor eingeleitet. Ca. 30 Minuten nach Ende des Einleitens wird mit Stickstoff ausgeblasen und dann über eine Füllkörperkolonne destilliert. Nach einem kleinen Vorlauf gehen 760 g Benzylchlorid (Kp: 114–118 °C/16 mm) über. Nach einem Zwischenlauf erhält man noch 200 g Benzalchlorid (Kp. 127 °C/16 mm).

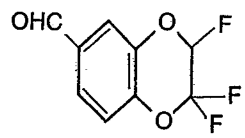

6-Formyl-trifluorbenzodioxen

Man löst 190 g Urotropin in 250 ml Wasser bei 100 °C und tropft 190 g Mischung aus Benzyl-/Benzalchlorid zu. Nach 1 Stunde bei 100 °C wird eine Mischung aus 200 ml Wasser und 200 ml Salzsäure zugegeben und anschliessend nochmals für 2 Stunden bei 100 °C gerührt. Dann wird das Produkt mit Wasserdampf übergetrieben und redestilliert. Ausbeute: 133 g 6-Formyltrifluorbenzodioxen.

## Patentansprüche

1. Substituierte Styrylcyclopropancarbonsäureester der Formel (I)

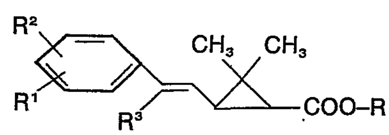 (I)

in welcher

R für den Rest eines gegebenenfalls substituierten Phenoxybenzylalkohols der allgemeinen Formel (III)

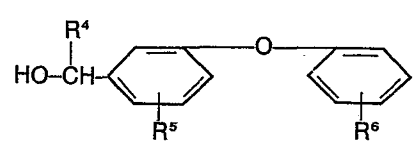 (III)

steht, worin $R^4$ für Wasserstoff, Cyano oder Ethinyl steht und $R^5$ und $R^6$ für Wasserstoff oder Fluor stehen,

$R^1$ für Alkoxy oder Alkylthio, die gegebenenfalls durch Halogen substituiert sind, steht,

$R^2$ für Wasserstoff, Alkoxy oder gemeinsam mit $R^1$ für gegebenenfalls halogensubstituiertes Alkylendioxy steht, und

$R^3$ für Wasserstoff oder Chlor steht.

2. Verfahren zur Herstellung von Styrylcyclopropancarbonsäureestern der Formel (I)

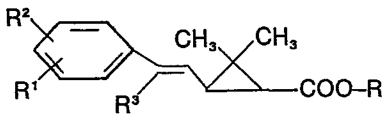

(I)

in welcher

R für den Rest eines gegebenenfalls substituierten Phenoxybenzylalkohols der Formel (III)

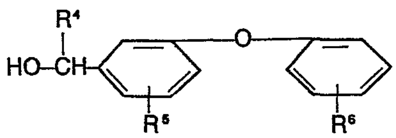

(III)

steht, worin

$R^4$ für Wasserstoff, Cyano oder Ethinyl steht und $R^5$ und $R^6$ für Wasserstoff oder Fluor stehen,

$R^1$ für Alkoxy oder Alkylthio, die gegebenenfalls durch Halogen substituiert sind, steht,

$R^2$ für Wasserstoff, Alkoxy oder gemeinsam mit $R^1$ für gegebenenfalls halogensubstituiertes Alkylendioxy steht, und

$R^3$ für Wasserstoff oder Chlor steht, dadurch gekennzeichnet, dass man Styrylcyclopropancarbonsäuren der Formel II

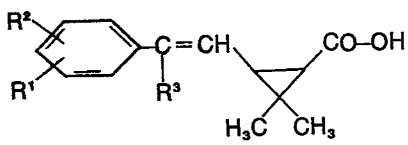

(II)

in welcher

$R^1$, $R^2$ und $R^3$ die oben angegebene Bedeutung haben, oder reaktionsfähige Derivate derselben, mit Alkoholen der Formel

R–OH

in welcher

R die oben angegebene Bedeutung besitzt, oder deren reaktionsfähige Derivate umsetzt.

3. Styrylcyclopropancarbonsäuren und deren niedere Alkylester der Formel (II)

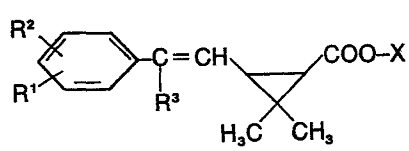

(II)

in welcher

$R^1$ für halogensubstituiertes Alkoxy steht,

$R^2$ für Wasserstoff steht,

$R^3$ für Wasserstoff oder Chlor steht und

X für Wasserstoff oder $C_{1-4}$-Alkyl steht.

4. Insektizide und/oder akarizide Mittel, gekennzeichnet durch einen Gehalt an mindestens einem substituierten Styrylcyclopropancarbonsäureester der Formel (I) gemäss Anspruch 1.

5. Verwendung von substituierten Styrylcyclopropancarbonsäureester der Formel (I) gemäss Anspruch 1 zur Bekämpfung von Insekten und/oder Spinnentieren.

6. Verfahren zur Herstellung insektizider und/oder akarizider Mittel, dadurch gekennzeichnet, dass man substituierte Styrylcyclopropancarbonsäureester der Formel (I) gemäss Anspruch 1 mit Streckmitteln und/oder oberflächenaktiven Mitteln vermischt.

**Claims**

1. Substituted styrylcyclopropanecarboxylic acid esters of the formula (I)

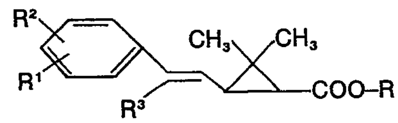

(I)

in which

R represents the radical of an optionally substituted phenoxybenzyl alcohol of the general formula (III)

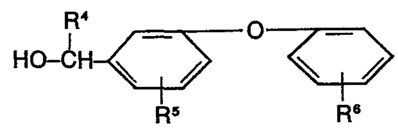

(III)

wherein

$R^4$ represents hydrogen, cyano or ethinyl and

$R^5$ and $R^6$ represent hydrogen or fluorine,

$R^1$ represents alkoxy or alkylthio, which are optionally substituted by halogen,

$R^2$ represents hydrogen or alkoxy or together with $R^1$, optionally halogen-substituted alkylenedioxy and

$R^3$ represents hydrogen or chlorine.

2. Process for the preparation of styrylcyclopropanecarboxylic acid esters of the formula (I)

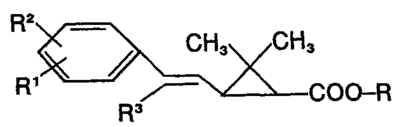

(I)

in which

R represents the radical of an optionally substituted phenoxybenzyl alcohol of the formula (III)

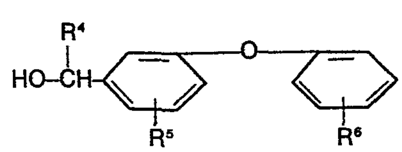

(III)

wherein

$R^4$ represents hydrogen, cyano or ethinyl and

$R^5$ and $R^6$ represent hydrogen or fluorine,

$R^1$ represents alkoxy or alkylthio, which are optionally substituted by halogen,

$R^2$ represents hydrogen or alkoxy or together with $R^1$, optionally halogen-substituted alkylenedioxy and

$R^3$ represents hydrogen or chlorine, characterised in that styrylcyclopropanecarboxylic acids of the formula II

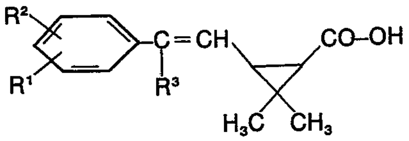

(II)

in which

$R^1$, $R^2$ and $R^3$ have the meaning indicated above, or reactive derivatives thereof, are reacted with alcohols of the formula

R–OH

in which

R has the meaning indicated above, or reactive derivatives thereof.

3. Styrylcyclopropanecarboxylic acids and their lower alkyl esters of the formula (II)

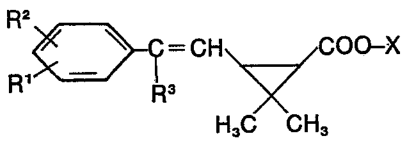

(II)

in which

$R^1$ represents halogen-substituted alkoxy,

$R^2$ represents hydrogen,

$R^3$ represents hydrogen or chlorine and

X represents hydrogen or $C_{1-4}$-alkyl.

4. Insecticidal and/or acaricidal agents, characterised in that they contain at least one substituted styrylcyclopropanecarboxylic acid ester of the formula (I) according to Claim 1.

5. Use of substituted styrylcyclopropanecarboxylic acid esters of the formula (I) according to Claim 1 for combating insects and/or arachnidae.

6. Process for the preparation of insecticidal and/or acaricidal agents, characterised in that substituted styrylcyclopropanecarboxylic acid esters of the formula (I) according to Claim 1 are mixed with extenders and/or surface-active agents.

**Revendications**

1. Esters d'acides styrylcyclopropanecarboxyliques substitués, caractérisés en ce qu'ils répondent à la formule (I):

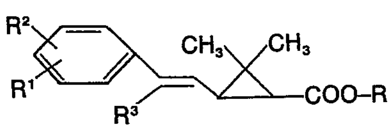

(I)

dans laquelle:

R représente le radical d'un alcool phénoxybenzylique éventuellement substitué, de formule générale (III)

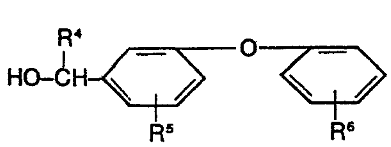

(III)

dans laquelle $R^4$ représente un atome d'hydrogène, un groupe cyano ou éthynyle et $R^5$ et $R^6$ représentent de l'hydrogène ou du fluor,

$R^1$ représente un groupe alcoxy ou alkylthio, qui peuvent éventuellement être substitués par de l'halogène,

$R^2$ représente un atome d'hydrogène ou un groupe alcoxy ou représente, pris avec $R^1$, un groupe alkylènedioxy éventuellement substitué par de l'halogène, et

$R^3$ est un atome d'hydrogène ou de chlore.

2. Procédé de production d'esters d'acides styrylcyclopropanecarboxyliques de formule (I)

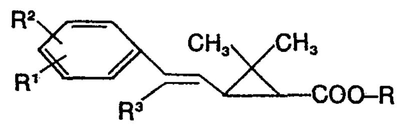

(I)

dans laquelle

R représente le radical d'un alcool phénoxybenzylique éventuellement substitué, de formule (III)

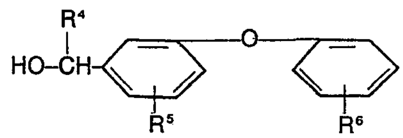

(III)

dans laquelle $R^4$ représente un atome d'hydrogène ou un groupe cyano ou éthynyle et $R^5$ et $R^6$ représentent chacun un atome d'hydrogène ou de fluor,

$R^1$ représente un groupe alcoxy ou alkylthio, qui sont éventuellement substitués par de l'halogène,

$R^2$ représente un atome d'hydrogène ou un groupe alcoxy ou, pris avec $R^1$, représente un groupe alkylènedioxy éventuellement substitué par de l'halogène, et

$R^3$ représente un atome d'hydrogène ou de chlore, procédé caractérisé en ce qu'on fait réagir les acides styrylcyclopropanecarboxyliques de formule (II)

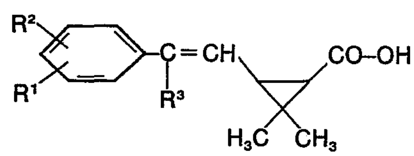

(II)

dans laquelle $R^1$, $R^2$ et $R^3$ ont le sens indiqué ci-dessus, ou des dérivés réactifs de ces acides, avec des alcools de formule

R–OH

dans laquelle R a le sens indiqué ci-dessus, ou avec leurs dérivés réactifs.

3. Acides styrylcyclopropanecarboxyliques et leurs esters alkyliques inférieurs, caractérisés en ce qu'ils répondent à la formule (II)

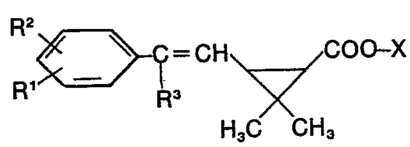

(II)

dans laquelle

$R^1$ représente un groupe alcoxy substitué par de l'halogène,

$R^2$ représente un atome d'hydrogène,

$R^3$ représente un atome d'hydrogène ou de chlore, et

X représente un atome d'hydrogène ou un groupe alkyle ayant 1 à 4 atomes de carbone.

4. Insecticides et/ou acaricides, caractérisés en ce qu'ils contiennent au moins un ester d'acide styrylcyclopropanecarboxylique substitué, répondant à la formule (I) selon la revendication 1.

5. Application d'esters d'acides styrylcyclopropanecarboxyliques substitués de formule (I) selon la revendication 1 à la lutte contre des insectes et/ou des acariens.

6. Procédé de production d'insecticides et/ou acaricides, caractérisé en ce qu'on mélange des esters d'acides styrylcyclopropanecarboxyliques substitués de formule (I) selon la revendication 1 avec des agents d'allongement et/ou des agents tensioactifs.